# EUROPÄISCHE PATENTSCHRIFT

(11) **EP 0 692 538 B1**
(45) Veröffentlichungstag und Bekanntmachung des Hinweises auf die Patenterteilung: **22.09.1999**
(21) Anmeldenummer: 95110765.5
(22) Anmeldetag: 11.07.1995
(51) Int. Cl.: C12P 13/04, C12P 41/00, C07C 229/08, C07C 227/16, C07C 227/32

(54) **Verfahren zur Herstellung optisch aktiver L-Aminosäuren**
Process for producing optically active L-amino acids
Procédé pour la préparation des acides L-aminés optiquement actifs

(30) Priorität: 15.07.1994 DE 4425068
(43) Veröffentlichungstag der Anmeldung: 17.01.1996
(73) Patentinhaber: Degussa-Hüls Aktiengesellschaft, 60287 Frankfurt am Main (DE)
(72) Erfinder: Bommarius, Andreas, Dr., D-60323 Frankfurt (DE); Drauz, Karlheinz, Prof. Dr., D-63579 Freigericht (DE); Krix, Georg, D-50679 Köln (DE); Kula, Maria-Regina, Prof. Dr., D-52382 Niederziehr (DE); Schwarm, Michael, Dr., D-63755 Alzenau (DE)

(56) Entgegenhaltungen:
- WO-A-95/11296
- DE-A- 1 417 030
- CHINESE CHEMICAL LETTERS, Bd. 3, Nr. 4, 1992, Seiten 237-238, XP002021201 SHEN ET AL.: "Optically Active Amino Acids with Highly Branched Side Chain (I)"
- CHINESE CHEMICAL LETTERS, Bd. 5, Nr. 5, 1994, Seiten 373-374, XP002021202 ZHANG ET AL: "Optically Active Amino Acids with Highly Branched Side Chain (II)"
- TETRAHEDRON LETT., Bd. 31, Nr. 42, 1990, Seiten 6005-6008, XP002021203 LOWENTHAL ET AL: "Asymmetric Catalytic Cyclopropanation of Olefins: Bis-Oxazoline Copper Complexes"
- ANG. CHEMIE, Bd. 99, Nr. 11, 1987, Seiten 1197-1199, XP002021204 EVANS ET AL: "Pi-Solvation von aromatischen Ringen im Übergangszustand"
- J.AM.CHEM.SOC., Bd. 110, 1988, Seiten 1238-1256, XP002021205 EVANS ET AL: "Asymmetric Diels-Alder Cycloaddition Reactions with Chiral alpha-beta-Unsaturated N-Acyloxazolidinones"
- TETRAHEDRON, Bd. 48, Nr. 13, 1992, Seiten 2589-2612, XP002021206 MEYERS A.I.: "Recent Progress Using Chiral Formamidines in Asymmetric Syntheses"
- ANG. CHEMIE, Bd. 103, Nr. 5, 1991, Seiten 556-558, XP002021207 BOLM C.: "Bis(4,5-dihydrooxazolyl)-Derivate in der asymmetrischen Katalyse"

## Beschreibung

Die Erfindung beschreibt ein Verfahren zur Herstellung optisch aktiver L-Aminosäuren der allgemeinen Formel II 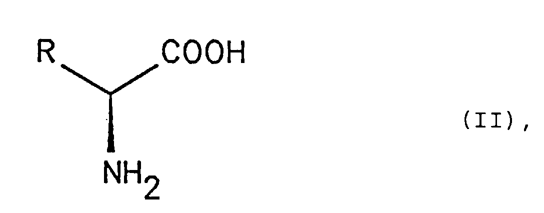 worin R für eine raumerfüllende, verzweigte, ein tertiäres C-Atom enthaltende Alkylgruppe mit 5 - 10 C-Atomen steht.

Optisch aktive Aminosäuren können zur Herstellung einer Reihe wertvoller Verbindungen eingesetzt werden. So ist grundsätzlich die Reduktion zu den entsprechenden optisch aktiven Aminoalkoholen bekannt. Diese wiederum sind äußerst wichtige Zwischenprodukte in der organischen Chemie. Sie oder von diesen abgeleitete Verbindungen finden breite Anwendung in der asymmetrischen Synthese, bei der Herstellung von pharmazeutischen Wirkstoffen wie zum Beispiel Peptiden, der Synthese von Isektiziden, der Spaltung racemischer Gemische und auf anderen Gebieten (weiterführende Literatur zu diesen Themen unter anderem in: J. Org. Chem. 1993, 58, 3568).

Der Einsatz von optisch aktiven Aminoalkoholen der allgemeinen Formel 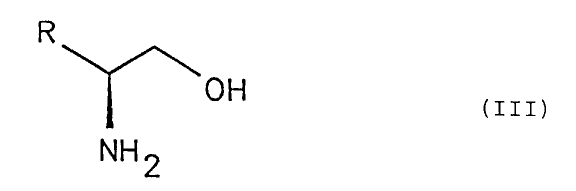 in der asymmetrischen Synthese beruht sehr häufig darauf, daß Aminoalkohole mit sterisch anspruchsvollen Seitengruppen in geeignete Derivate eingebaut werden, mit denen eine asymmetrische Katalyse oder asymmetrische Induktion bei der Synthese von Folgeprodukten möglich ist. Beispiele für derartige Aminoalkohole sind unter anderem Phenylglycinol (R = Ph), Phenylalaninol (R = CH₂Ph), Valinol (R = CHMe₂) und tert.-Leucinol (R = CMe₃). Diese können beispielsweise zu optisch aktiven Oxazolinen und ähnlichen Verbindungen derivatisiert werden, die wiederum als Liganden für hochwirksame Katalysatoren eingesetzt werden können, zum Beispiel in der asymmetrischen Cyclopropanierung und Reduktion von Olefinen, der Hydrosilylierung und Reduktion von Ketonen, bei Diels-Alder-Reaktionen und nucleophilen Substitutionen (weiterführende Literatur z. B. in: Angew. Chem. 1991, 103, 556). Als Beispiele für in der asymmetrischen Synthese vielseitig einsetzbare Folgeprodukte der optisch aktiven Aminoalkohole seien 4-substituierte 2-Oxazolidinone (Org. Synth. 1989, 68, 77 und dort zitierte Lit.), bicyclische Lactame (Tetrahedron 1991, 47, 9503 und dort zitierte Lit.) und Formamidine (Tetrahedron 1992, 48, 2589 und dort zitierte Lit.) genannt.

In den genannten und vielen weiteren Fällen üben die Seitengruppen der eingebauten optisch aktiven Aminoalkohole der allgemeinen Formel (III) aus sterischen oder stereoelektronischen Gründen einen dirigierenden Einfluß auf die an und mit diesen Molekülen ablaufenden Reaktionen aus, woraus sich die teilweise außerordentlich hohen Enantio- oder Diastereoselektivitäten solcher Folgereaktionen ergeben. Dieser dirigierende Einfluß ist in vielen Fällen umso größer, je raumerfüllender die genannten Seitengruppen R sind (Angew. Chem. 1991, 103, 556). So ist der erzielte e.e. oder d.e. häufig größer, wenn die Seitengruppe ein tert.-Butylrest (R = CMe₃) anstelle eines Isopropylrestes (R = CHMe₂) ist (Beispiele siehe unter anderem: Tetrahedron Lett. 1990, 31, 6005; Tetrahedron 1992, 48, 2589; Angew. Chem. 1987, 99, 1197; J. Am. Chem. Soc. 1988, 110, 1238).

In Chinese Chemical Letters 1994, 5, 373-374 und Chinese Chemical Letters 1992, 3, 237-238 ist ein Verfahren zur Herstellung von sterisch anspruchsvollen Aminosäuren genannt.

Die DE 14 17 030 beschreibt ein Verfahren zur biotechnischen Herstellung von L-Glutaminsäure aus Zuckern. Innerhalb der metabolischen Umwandlung des Zuckers findet auch die Glutaminsäuredehydrogenase des eingesetzten Organismus Anwendung.

In der WO 95/11296, die zwischen Prioritätstag und Anmeldetag der vorliegenden Anmeldung veröffentlicht wurde, wird ein Verfahren zur Abänderung von Enzymen beschrieben. Mithin ist auch die Umsetzung von 2-Ketocaproat mittels einer veränderten Glutamatdehydrogenase beschrieben.

Angesichts dieser Tatsachen ist es Aufgabe der vorliegenden Erfindung, ein weiteres Verfahren zur Herstellung optisch aktiver Aminosäuren anzugeben, das die Bereitstellung von optisch aktiven L-Aminosäuren erlaubt, deren Reste R den sterischen Anspruch einer tert.-Butylgruppe nach Möglichkeit noch übersteigen, um so bei asymmetrischen Synthesen einen noch größeren dirigierenden Einfluß ausüben und damit die Enantio- und Diastereoselektivität bei diesen Reaktionen noch zu verbessern.

Diese Aufgaben werden nun erfindungsgemäß dadurch gelöst, daß α-Ketocarbonsäuren der allgemeinen Formel 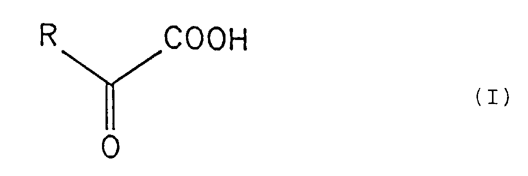 worin R jeweils für eine raumerfüllende, verzweigte, wenigstens ein tertiäres C-Atom enthaltende Alkylgruppe mit 5 - 10 C-Atomen steht, durch co-faktorabhängige enzymatische reduktive Aminierung unter Verwendung von Dehydrogenasen in die optisch aktiven L-Aminosäuren der allgemeinen Formel 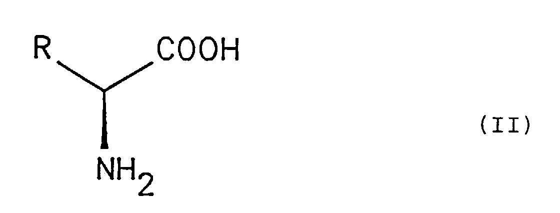 überführt werden, worin R die oben angegebene Bedeutung hat. Es war dabei insbesondere überraschend, keinesfalls vorhersehbar und erfindungsgemäß besonders vorteilhaft, daß die verwendeten Enzyme auch α-Ketosäuren der allgemeinen Formel (I) mit ihren besonders raumerfüllenden Resten R als Substrate zur Umwandlung in die L-Aminosäuren der allgemeinen Formel (II) akzeptieren und daß die Produkte auch noch mit hoher chemischer und Enantiomerenreinheit sowie in guter bis sehr guter Ausbeute erhalten werden.

Die Herstellung der in dieser Erfindung beschriebenen Verbindungen wird in folgendem Formelschema nochmals zusammengefaßt: 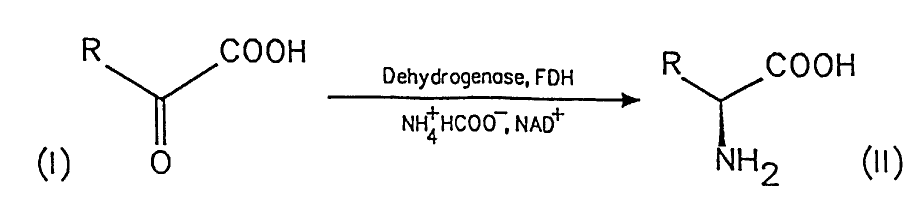 Insgesamt werden mit der vorliegenden Erfindung ein Verfahren zur Herstellung optisch aktiver L-Aminosäuren der allgemeinen Formel (II) zur Verfügung gestellt.

Erfindungsgemäß sind diese Verbindungen leicht, sicher, in guter bis sehr guter Ausbeute und in sehr hoher chemischer und insbesondere Enantiomerenreinheit herstellbar. Die so hergestellten Verbindungen finden Einsatz unter anderem in der Synthese von Pharmawirkstoffen und in der asymmetrischen Synthese zur Synthese von Liganden, Katalysatoren und Induktoren.

Die erfindungsgemäßen Verbindungen sowie die Verfahren zu ihrer Herstellung werden durch die nachfolgenden Beispiele näher erläutert:

### Beispiel 1: Darstellung von (S)-Neopentylglycin

31.53 g (0.5 mol) Ammoniumformiat und 20.89 g (125 mmol) 2-Keto-4,4-Dimethylpentansäure Na-Salz werden in 400 ml Wasser suspendiert, der pH-Wert mit Ammoniak auf 8.2 eingestellt, so daß eine Lösung vorliegt und auf 500 ml aufgefüllt. Anschließend werden 71.7 mg (0.1 mmol) NAD⁺·H₂O-Cofaktor sowie 2000 U an Leucindehydrogenase (LeuDH) und 2500 U an Formiatdehydrogenase (FDH) zugegeben. Es wird eine Temperatur von 28 °C eingestellt, während der Reaktion gelinde gerührt und der pH-Wert auf 8.2 durch eine pH-Statisierungseinrichtung konstant gehalten. Nach 48 Stunden wird durch Bestimmung des Umsatzes per HPLC nachgewiesen, daß die Reaktion beendet ist. Die Enzyme werden über ein Ultrafilter der Porenweite von 10 kDa abgetrennt und die Reaktionslösung mit Ammoniak auf pH 9.5 eingestellt. Anschließend wird mit 2 % Aktivkohle geklärt und die fast farblose Lösung am Rotationsverdampfer eingeengt, die Aminosäure auskristallisiert, über eine Nutsche abgetrennt, dreimal mit wenig Ethanol gewaschen und im Vakuum über Nacht bei 50 °C getrocknet.

| | |
|---|---|
| Ausbeute | 15.4 g (84.9 % der Theorie) |
| Identitätsprüfung | NMR-Spektrum |
| Enantiomerenreinheit | > 99.8 % e.e., gemessen durch chirale Gaschromatographie an Chirasil-Val |

### Beispiel 2: Darstellung von (S)-3-Methyl-Isoleucin ((S))-3,3-Dimethyl-norvalin)

6.3 g (0.1 mol) Ammoniumformiat und 1.67 g (10 mmol) 2-Keto-3,3-Dimethylpentansäure Na-Salz werden in 80 ml Wasser suspendiert, der pH-Wert mit Ammoniak auf 8.2 eingestellt, so daß eine Lösung vorliegt und auf 100 ml aufgefüllt. Anschließend werden 14.34 mg (0.02 mmol) NAD⁺·3 H₂O-Cofaktor sowie 800 U an Leucindehydrogenase (LeuDH) und 500 U an Formiatdehydrogenase (FDH) zugegeben. Es wird eine Temperatur von 32 °C eingestellt, während der Reaktion gelinde gerührt und der pH-Wert auf 8.2 durch eine pH-Statisierungseinrichtung konstant gehalten. Nach spätestens 72 Stunden kann durch Bestimmung des Umsatzgrades per HPLC nachgewiesen werden, daß die Reaktion beendet ist. Die Reaktionslösung wird mit Ammoniak auf pH 9.5 eingestellt und anschließend mit 2 % Aktivkohle geklärt. Die fast farblose Lösung wird am Rotationsverdampfer eingeengt, die Aminosäure auskristallisiert, über eine Nutsche abgetrennt, dreimal mit wenig Ethanol gewaschen und im Vakuum über Nacht bei 50 °C getrocknet.

| | |
|---|---|
| Ausbeute | 1.17 g (80.6 % der Theorie) |
| Identitätsprüfung | NMR-Spektrum |
| Enantiomerenreinheit | > 99.9 % e.e., gemessen durch chirale Gaschromatographie an Chirasil-Val |

### Beispiel 3: Darstellung von (S)-Homoneopentylglycin ((S)-5,5-Dimethyl-norleucin)

Reaktion und Aufarbeitung wurden analog zu Beispiel 4 durchgeführt, mit der Ausnahme, daß während der gesamten Reaktionszeit eine Suspension vorlag und die Reaktion erst nach 96 Stunden beendet war.

| Einsatz: 1.81 g (10 mmol) 2-Keto-5,5-Dimethyl-hexansäure Na-Salz | |
|---|---|
| Ausbeute | 1.08 g (67.9 % der Theorie) |
| Identitätsprüfung | NMR-Spektrum |
| Enantiomerenreinheit | > 99.9 % e.e., gemessen durch chirale HPLC an Crownpak-CR+Säule |

## Patentansprüche

1. Verfahren zur Herstellung optisch aktiver L-Aminosäuren der allgemeinen Formel II 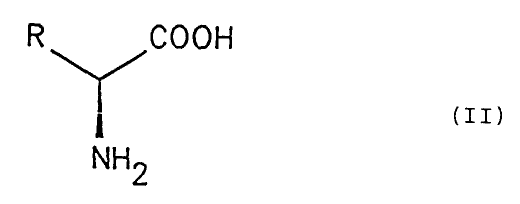 worin R für eine raumerfüllende, verzweigte, wenigstens ein tertiäres C-Atom enthaltende Alkylgruppe mit 5-10 C-Atomen steht,
**dadurch gekennzeichnet**,
daß eine α-Ketocarbonsäure der allgemeinen Formel I 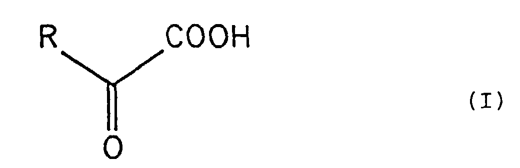 worin R für eine raumerfüllende, verzweigte, wenigstens ein tertiäres C-Atom enthaltende Alkylgruppe mit 5 - 10 C-Atomen steht, durch eine co-faktorabhängige enzymatische Reaktion unter Verwendung von Dehydrogenasen reduktiv aminiert wird.

## Claims

1. Process for the preparation of optically active L-amino acids corresponding to the general formula II 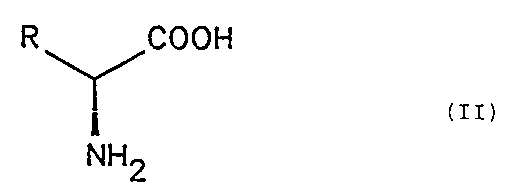 wherein R represents a space-filling, branched alkyl group having 5-10 C atoms and containing at least one tertiary C atom,
characterised in that
an α-ketocarboxylic acid corresponding to the general formula I 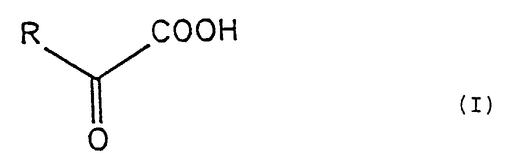 wherein R represents a space-filling, branched alkyl group having 5-10 C atoms and containing at least one tertiary C atom, is reductively aminated by a cofactor-dependent enzymatic reaction with the use of dehydrogenases.

## Revendications

1. Procédé de préparation d'acides L-aminés optiquement actifs de formule générale II : 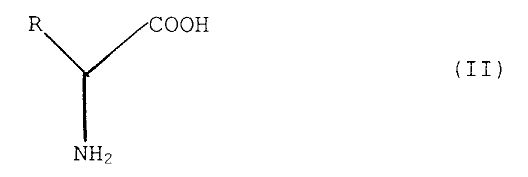 dans laquelle R désigne un groupe alkyle dense ramifié de 5-10 atomes de carbone contenant au moins un atome de carbone tertiaire,
caractérisé en ce qu'un acide α-cétocarboxylique de formule générale I : 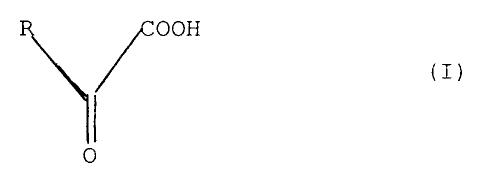 dans laquelle R désigne un groupe alkyle dense ramifié de 5-10 atomes de carbone contenant au moins un atome de carbone tertiaire, est aminé dans des conductions réductrices par une réaction enzymatique fonction d'un co-facteur en utilisant des déshydrogénases.
